# EUROPEAN PATENT APPLICATION

(11) **EP 3 564 672 A1**
(43) Date of publication of application: **06.11.2019**
(21) Application number: 16925045.3
(22) Date of filing: 28.12.2016
(51) Int. Cl.: G01N 33/53, G01N 33/543, A61M 37/00, A61B 5/00, C12N 15/115

(54) **METHOD FOR FABRICATING MICRONEEDLE-BASED DIAGNOSTIC SKIN PATCH COATED WITH APTAMER AND PATCH**

(71) Applicant: Nexmos Co., Ltd., Seoul (KR)
(72) Inventor: SON, In Sik, Seongnam-si Gyeonggi-do 13614 (KR)
(74) Representative: Berggren Oy, Helsinki & Oulu
(86) International application number: PCT/KR2016/015395
(87) International publication number: WO 2018/124327

(57) **Abstract**

The present invention relates to a method for fabricating an aptamer-coated, microneedle-based diagnostic skin patch and a patch fabricated thereby. A patch of the present invention has the advantage of attaching a great number of aptamers, which are much smaller in size than antibodies, onto a relatively great number of microneedle tip surfaces, Allowing the attachment of aptamers for various kinds of biomarkers all together thereto, the patch can also simultaneously detect various kinds of materials (multiplexing). Therefore, a microneedle tip-based skin patch can also be used as a protein chip using aptamer.

## Description

### [Technical Field]

The present invention relates to a method for fabricating a microneedle-based diagnostic skin patch coated with an aptamer and a patch fabricated by such method.

### [Background Art]

skin diseases represent a major healthcare challenge in the world today. Predicting and diagnosing a skin disease are important for its management, together with more than one million new skin cancers diagnosed each year in US (Nation Cancer Institute). Current diagnostic methods rely mainly on visual observation and biopsy. However, detection methods depending on visual observation are not necessarily effective in diagnosing skin conditions or diseases, and do not detect any risk or diseases until clinical manifestations occur. Furthermore, invasive methods such as biopsy increase the likelihood of infections as well as trauma to the test subject. In addition, the method should be performed by a physician in order to be safely performed, and usually does not provide a cell sample being rich on the skin surface, which is the cell generally involved in the response.

Therefore, non-invasive method for diagnosing and monitoring skin conditions and diseases represent an important means for patient management and for evaluating the efficacy of existing and new treating agents, skin care products and skin care regimens. Furthermore, the method can provide important information about specific genetic changes based on the skin condition of the test subject, as well as the genetic susceptibility of the test subject to an occurrence of skin disease. Identifying the genetic alteration may be important in identifying potential drug targets and preventative measures and determining whether a person actually responds to a particular therapeutic agent, skin care product or regimens. In addition, detection and diagnostic methods are important in assessing the safety of such treatments, products and measures.

Moreover, it has been reported that the composition of skin material changes in various disease states as well as local skin disease. Various substances, such as lipids, structural proteins, inflammatory substances, nucleic acids, metabolites, etc. are known to be variously detected in the skin depending on the disease state. At present, in addition to atopic dermatitis, melanoma, and bacterial inflammation of the skin, a biomarker analysis of skin has been performed in various diseases such as Alzheimer's disease, Parkinson's disease, breast cancer, cardiovascular disease, diabetes, drug addiction and the like. In most cases, however, a very invasive skin biopsy is being used. Although Montophoresis, Microdialysis, Tape stripping, Ultrasound, Microneedle and the like are used as non-invasive methods, their efficiencies are regarded as being very low (Paliwal et al., 2013 Diagnostic opportunities based on skin biomarkers. European journal of pharmaceutical sciences : official journal of the European Federation for Pharmaceutical Sciences 50:546-556).

### [Patent Document of Prior Art]

Korean Laid-open Patent Publication No. 1020120006945.

### [Disclosure]

### [Technical Problem]

The present invention has been made in view of needs to resolve the above problems, and an object of the present invention is to provide a method for manufacturing a patch for use in diagnosis of various diseases.

Another object of the present invention is to provide a patch for use in diagnosis of various diseases.

### [Technical Solution]

In order to accomplish the above object, the present invention provides a method for fabricating a microneedle-based diagnostic skin patch coated with an aptamer, comprising steps for sequentially coating a polycarbonate microneedle with titanium and gold to prepare a coated polycarbonate microneedle; preparing an aptamer bonded to polyethylene glycol (PEG) having a thiol group; and coupling the polycarbonate microneedle coated with titanium and gold with the aptamer bonded to polyethylene glycol (PEG) having a thiol group.

In one embodiment of the present invention, the thickness of the coated gold is preferably 10 to 40 nm, but is not limited thereto.

In addition, the present invention provides to a method for fabricating a microneedle-based diagnostic skin patch coated with an aptamer, comprising steps for a) binding an amine group to the aptamer; b) after attaching silanol on a surface of the microneedle tip with plasma oxidation, silanizing hydroxyl group with 3-glycidoxypropyltrimethoxysilane (3-GPTMS), and then, binding the amine group bonded to the aptamer to epoxy group contained in the 3-GPTMS.

Further, the present invention provides a method for manufacturing a microneedle-based diagnostic skin patch, comprising the steps for treating NHO₃ on the surface of the polycarbonate microneedle; reducing it with NaBH4; finally binding amine group to it; and then binding carboxyl group on one end and polyethyleneglycol (PEG) bonded with the aptamer by using NHS(N-Hydroxysuccinimide), and EDC[1-ethyl-3-(3'-dimethylaminopropyl)carbodiimide]as the catalysts.

The present invention also provides a microneedle-based diagnostic skin patch coated with the aptamer prepared by the method of the present invention.

In one embodiment of the present invention, the diagnostic skin patch is characterized in that the aptamer is attached on the surface of the microneedle tip and the diagnosable component can be attached to the end of the aptamer, but is not limited thereto. The diagnosable component is preferably a protein, a peptide, DNA or RNA, but is not limited thereto.

Hereinafter, the present invention will be described.

The present invention relates to a diagnostic skin patch which effectively detect various biomarkers existing in the cellular interstitials in the skin by coating the surface of a microneedle having a length of about 200 um which can penetrate the stratum corneum of the skin and reach epidermis, with aptamer.

The kinds of biomarker that is contained in the cellular interstitials of the skin and thus can be captured are the pathogen itself in various infectious diseases or the substance secreted by the pathogen, and is for example, malaria pathogen itself in the case of malaria or a protein secreted by the pathogen, etc.; the representative protein is PfHRP2; and a virus itself in the case of various virus can be detected. In addition, for various degenerative brain diseases, for example, Tau protein is increased in Parkinson's disease or Alzheimer's disease, and thus can also be used as a marker; Alpha-synuclein can be used as the important diagnostic marker for Parkinson's disease, Alymoid beta can be used as the diagnostic marker for Alzheimer's disease; and also various cancer labelling agents can be used as a biomarker which can be captured.

In relation to a manufacture of the microneedle coated with aptamer of the present invention, various microneedles have been developed for the delivery of effective drugs through brain machine interfaces (BMIs) or skin, and have been tested for measuring the brain wave, etc. through the scalp.

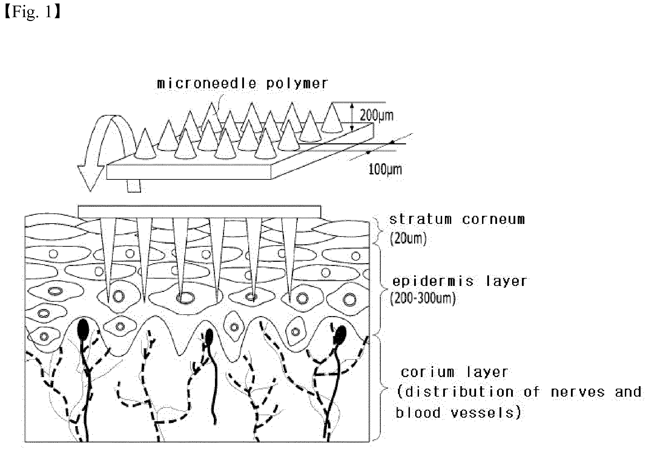

As an example, upon referring to the paper, Ami, Y et al, J of Micro/Nanolithography, MEMS, and MOEMS. 10 (1), 011503, March 2011, as published on 2011, it can be seen that the microneedle for penetrating the skin with a thickness of 50 um and a length of 200 um has been successfully developed by using polymethlsiloxane (PDMS) as a raw material (FIG. 1).

In addition to this, microneedles (microneedle or microprojection array) with various raw material and length have been manufactured. The kinds of chemicals used as the raw material in microneedle are as follows: Polyurethane (PU), Polypropylene (PP), Polyethylene (PE), Polystyrene (PS), Poly(methyl methacrylate) (PMMA), Polydimethylsiloxane (PDMS), Polycarbonate (PS), Liquid crystal polymer (LCP).

An aptamer is a method for detecting a specific substance using a three-dimensional structure of single strand DNA or RNA, and has an advantage that it is similar to an antigen-antibody reaction but the size of the substance is so small that a large number of aptamers can be bound to the end of the microneedle or the inner hole.

Further, aptamers for several kinds of biomarkers can be attached at once (a concept similar to a gene chip), so that many kinds of substances can be detected at the same time (Multiplexing). In the case of a gene chip, it can combine genomics that can detect more than 10,000 kinds of DNA or RNA on a nail-size chip, and thus, theroretically, skin patch can also be used as a biomarker chip by activating the microneedle with the aptamer for various substances.

If a diagnostic skin patch is developed by using an aptamer, since biomarkers present in the cellular interstitials of the epidermal layer are to be detected, there is a problem that it must be penetrate about 20 um of stratum cornea. In order to manufacture a diagnostic skin patch in which the said problem can be resolved through microneedle, the aptamer can be attached to the microneedle, as follows:

### [Advantageous Effects]

As can be seen from the present invention, by the method for detecting the specific substance using a three-dimensional structure of a single strand DNA or RNA, called as the aptamer in the present invention, there is an advantage that a large number of aptamers can be combined on the surface of the tips of the microneedle wherein the substances have much smaller size and relatively large numbers, although it is similar to an antigen-antibody reaction.

In addition, since aptamer for various kinds of biomarkers can attached at the same time, various kinds of substance can be detected (Multiplexing), and thus, microneedle tip-based skin patch can be used as a protein chip using the aptamer.

### [Description of Drawings]

**FIG. 1** depicts a manufacture of a microneedle by using PDMS and the structure of the microneedle matched with layers of the skin.

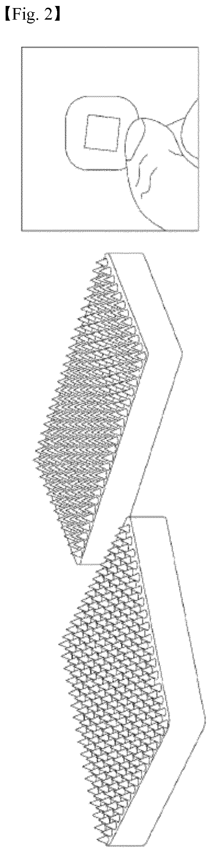

**FIG. 2** depicts a microneedle and an example of a patch using it.
**FIG. 3** depicts a flow chart of the method comprising steps for attaching silanol to the surface of tip of microneedle by plasma oxidation, silanizing it on hydroxyl group by 3-GPTMS, and binding amine group of PEG bonded with the aptamer to epoxy group contained in 3-GPTMS.
**FIG. 4** depicts an aptamer connected to PEG to which amine (NH2-) group is bonded.

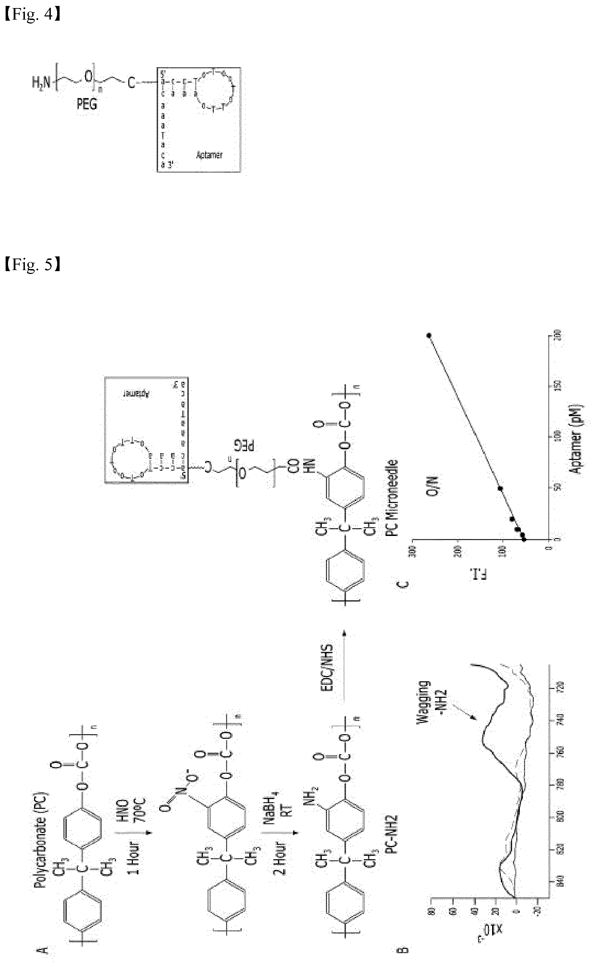

**FIG. 5** depicts a method for coating the surface of polycarbonate microneedle with the aptamer bonded on one end of PEG. (A) The surface of PC microneedle is treated with HNO₃ and reduced with NaBH₄ to finally bind the amine group. Thereafter, PEG in which carboxyl group is bonded to one end and aptamer is bonded to another end is treated to PC-NH2, together with EDC/NHS to complete PC microneedle in which the completed PECT-aptamer is functionalized. (B) PC to which NH2- is bonded through ATR-FTIR is identified. (C) Amounts of the aptamer attached to the surface of PC are analyzed using fluorescence tagged anti-sense oligonucleotide.
**FIG. 6** depicts PC microneedle the surface of which is coated with PEG-aptamer.
**FIG. 7** depicts an example using gold coated polycarbonate microneedle, wherein PC microneedle is sequentially coated by a 10 nm thick titanium layer and a 40 nm thick gold layer, and then, PEG-aptamer is bonded to the surface of the microneedle through a thiol-gold reaction.
**FIG. 8** depicts the results obtained by testing the number of molecules and stability at room temperature for PEC aptamer coated to PC microneedle through thiol-gold bond for about 40 days, and it could be identified that the number of the bonded molecules in PEG-aptamer increased in proportion to the thickness of gold coating, and the bond was stably maintained at room temperature for a long period of time.

### [Mode for Invention]

The present invention will now be described in more detail by way of non-limiting examples. Provided that, the following examples are intended to illustrate the present invention and the scope of the present invention is not to be construed as being limited by the following examples.

### Example 1: Example of reaction attaching an aptamer to the surface of the Microneedle tip

To avoid inaccuracy in diagnosis due to the proteins nonspecifically binding to the microneedle, the surface of microneedle is treated with polyethylene glycol (PEG) and then the aptamer is attached to the end of the PEG to construct the instrument.

### A. Example of PDMS microneedle:

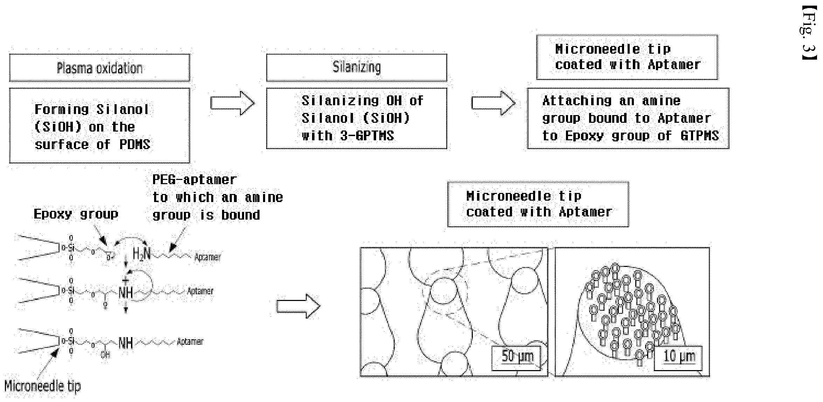

Silanol (SiOH) is formed by plasma oxidation of the surface of PDMS constituting a microneedle. This hydroxyl group is silanized with 3-glycidoxypropyltrimethoxysilane (3-GPTMS) and then the amine group of PEC bounded with the aptamer (FIG. 4) is combined to the expoxy group to constitute aptamer-PEG on the surface of microneedle (FIG. 3).

### B. Method for coating the surface of polycarbonate microneedle with aptamer bounded to one end of PEG:

The surface of PC microneedle is subjected to electrophilic substitution by treating it with nitric acid to reduce nitro group and attach primary amine group to prepare the nitro group PC-NH2. An aptamer bound to PEG with a carboxyl group is coupled with N-Hydroxysuccinimide (NHS), 1-ethyl-3-(3'-dimethylaminopropyl)carbodiimide (EDC) as a catalyst (FIG. 5A). That is, the surface of PC microneedle is treated with HNO3 and then reduced with NaBH4 to finally bind the amine group. Thereafter, PEG wherein a carboxyl group is bound on one end and the aptamer is bound on another end is treated to PC-NH2, together with EDC/NHS to complete PC microneedle in which the completed PEG-aptamer is functionalized.

The binding of the amine group to the polycarbonate was confirmed by ATR-FTIR method (FIG. 5B), and the binding of aptamer bound to PEG by the second reaction was verified by a method using a fluorescence tagged oligonucleotide (FIG. 5C).

Through the above two-step reaction, a biomarker diagnostic skin patch for skin in which the surface of the polycarbonate microneedle is coated with the aptamer attached to PEG was constructed.

### C. Example using Gold coated polycarbonate microneedle:

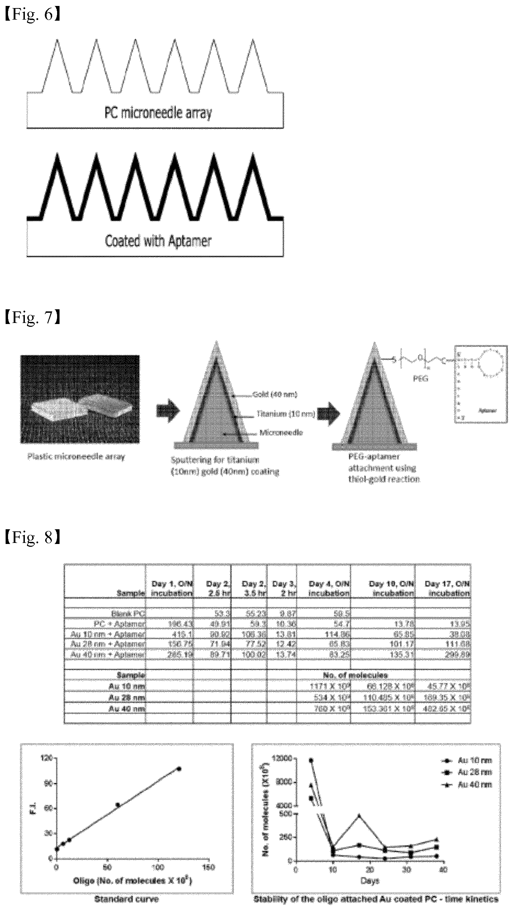

A diagnostic microneedle the surface of which is functionalized with DNA aptamer can be embodied through the method comprising steps for sequentially coating the surface of PC microneedle with titanium and gold through sputtering; reacting aptamer bound to PEG with the thiol group with Tris (2-carboxyethyl)phosphine hydrochloride (TCEP) at the room temperature for one hour; and couplng it with the prepared gold-coated PC microneedle at the room temperature for 24 hours (FIG. 7). The coating state of the surface was confirmed by scanning electron microscope (SEM).

As a result of testing the number of PEG-aptamer bound to the surface of PC microneedle and the binding stability at the room temperature, it was confirmed that about 2 x 10¹⁰ aptamers were stably bonded to the surface of the microneedle for more than 40 days (FIG. 8).

## Claims

1. A method for fabricating a microneedle-based diagnostic skin patch coated with an aptamer, comprising steps for sequentially coating a polycarbonate microneedle with titanium and gold to prepare a coated polycarbonate microneedle; preparing an aptamer bonded to polyethylene glycol (PEG) having a thiol group; and coupling the polycarbonate microneedle coated with titanium and gold with the aptamer bonded to polyethylene glycol (PEG) having a thiol group.

2. The method for fabricating microneedle-based diagnostic skin patch coated with aptamer, according to claim 1, **characterized in that** the thickness of the coated gold is 10 to 40 nm.

3. A method for fabricating a microneedle-based diagnostic skin patch coated with an aptamer, comprising steps for treating NHO₃ on the surface of the polycarbonate microneedle; reducing it with NaBH₄; finally binding amine group to it; and then coupling polyethyleneglycol (PEG) wherein carboxyl group is bound on one end and the aptamer is bound on another end with NHS(N-Hydroxysuccinimide), and EDC[1-ethyl-3-(3'-dimethylaminopropyl)carbodiimide] as the catalysts.

4. A microneedle-based diagnostic skin patch coated with the aptamer prepared by the method as defined in any one of claims 1 to 3.

5. The microneedle-based diagnostic skin patch, according to claim 4, **characterized in that** the aptamer is attached on the surface of the microneedle tip and the diagnosable component can be attached to the end of the aptamer.

6. The microneedle-based diagnostic skin, according to claim 4, **characterized in that** the diagnosable component is a protein, peptide, DNA or RNA.
